(19) Europäisches Patentamt / European Patent Office / Office européen des brevets

(11) EP 4 134 360 A1

(12) EUROPEAN PATENT APPLICATION
published in accordance with Art. 153(4) EPC

(43) Date of publication:
**15.02.2023 Bulletin 2023/07**

(21) Application number: **21784331.7**

(22) Date of filing: **25.03.2021**

(51) International Patent Classification (IPC):
***C07C 29/04*** *(1968.09)* ***C07C 31/08*** *(1968.09)*
***C07B 61/00*** *(1985.01)* ***B01J 23/30*** *(1974.07)*

(52) Cooperative Patent Classification (CPC):
(C-Sets available)
**B01J 27/19; B01J 21/08; C07B 61/00; C07C 29/04;**
Y02P 20/52 (Cont.)

(86) International application number:
**PCT/JP2021/012703**

(87) International publication number:
**WO 2021/205900 (14.10.2021 Gazette 2021/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.04.2020 JP 2020071176**

(71) Applicant: **Showa Denko K.K.**
**Tokyo 105-8518 (JP)**

(72) Inventors:
- **KOYANO, Masafumi**
  **Oita-shi, Oita 870-0189 (JP)**
- **KIMURA, Toshihiro**
  **Oita-shi, Oita 870-0189 (JP)**
- **INOUE, Gen**
  **Oita-shi, Oita 870-0189 (JP)**

(74) Representative: **Strehl Schübel-Hopf & Partner**
**Maximilianstrasse 54**
**80538 München (DE)**

# (54) METHOD FOR PRODUCING ALCOHOL

(57) A method for producing an alcohol by hydrating an olefin using a heteropolyacid catalyst is provided, in which an alcohol having high product purity can be stably produced over a long period of time. The method is for producing an alcohol by supplying a raw material mixture comprising water and an olefin having a carbon atom number of X, wherein X is an integer of 2 to 5, to a reactor and subjecting them to a hydration reaction in a gas phase using a solid acid catalyst on which a heteropolyacid or a salt thereof is supported to obtain a reaction product, wherein the content of an impurity olefin having a carbon atom number of Y, wherein Y is an integer of 2 to 6 and Y and X are different, contained in the raw material mixture is 700 mol ppm or less.

FIG. 1

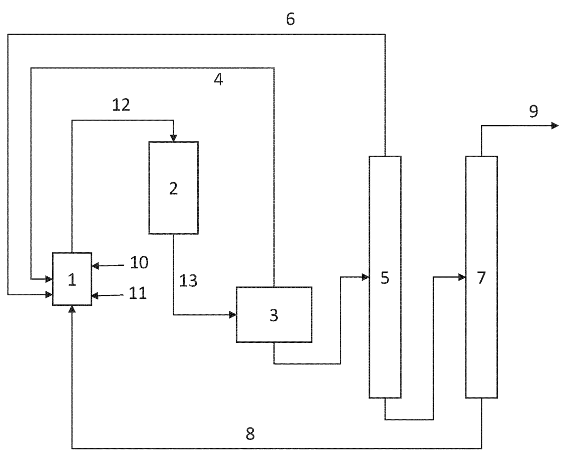


EP 4 134 360 A1

(52) Cooperative Patent Classification (CPC): (Cont.)

C-Sets
**C07C 29/04, C07C 31/08**

## Description

TECHNICAL FIELD

The present invention relates to a method for producing an alcohol by a hydration reaction of an olefin using a heteropolyacid catalyst. The present invention is particularly suitable for the production of ethanol from ethylene.

BACKGROUND ART

Industrial ethanol is an important industrial chemical product widely used as an organic solvent, an organic synthetic raw material, a disinfectant, and an intermediate of chemicals. It is known that industrial ethanol can be obtained by a hydration reaction of ethylene in the presence of a liquid acid, such as sulfuric acid and sulfonic acid, a zeolite catalyst, a metal oxide catalyst including tungsten, niobium or tantalum, etc., a heteropolyacid, such as phosphotungstic acid and silicotungstic acid, or a solid catalyst in which phosphoric acid is supported on a silica carrier or a diatomite carrier.

A hydration reaction of ethylene in a liquid phase using a liquid acid, such as sulfuric acid and sulfonic acid, as a catalyst requires a post-treatment of the acid used in the reaction, and in addition, is low in activity, so that industrial utilization thereof was limited. On the other hand, since a hydration reaction of ethylene using a solid catalyst of a carrier supported type can be carried out as a gas phase reaction, there is an advantage that the separation of a reaction product and the catalyst is easy, and the reaction can be carried out under a high temperature condition which is advantageous in terms of reaction kinetics or a high pressure condition which is advantageous in terms of the theory of equilibrium.

Regarding solid acid catalysts, many proposals have been made so far, and in particular, a gas phase reaction process using a solid acid catalyst in which phosphoric acid is supported on a carrier has already been industrially carried out. However, in this industrial process using a catalyst in which phosphoric acid is supported on a carrier, an efflux of phosphoric acid, which is an active component, continuously occurs, and as a result, the activity and selectivity decrease. Thus, continuous supply of phosphoric acid was required. Further, periodic maintenance of a reactor and other equipment is necessary, and thus this costs a lot to maintain the reactor and other equipment, since the effused phosphoric acid corrodes the equipment. In addition, a phosphoric acid supported catalyst is physically and chemically deteriorated by contacting with water vapor. For that reason, when the phosphoric acid supported catalyst was used for a long period of time, the activity thereof decreased, and in some cases, carrier particles aggregated with each other to form a block, so that it was extremely difficult to replace and extract the catalyst. Therefore, a novel carrier and a supported catalyst have been developed to solve these problems in the hydration reaction of ethylene.

As a catalyst for a hydration reaction of ethylene without a risk of an efflux of phosphoric acid, metal oxide catalysts are known, and a zeolite catalyst (Patent Literature 1), a metal oxide catalyst containing titanium oxide and tungsten oxide as essential components (Patent Literature 2), and a metal oxide catalyst containing tungsten and niobium as essential components (Patent Literature 3) are known. However, hydration reactions of ethylene using these metal oxide catalysts were less active than the case where a phosphoric acid catalyst was used, and the selectivity of the reaction was also low.

As another catalyst capable of avoiding an efflux of phosphoric acid, a solid acid catalyst in which a heteropolyacid is supported on a carrier is known. For example, a catalyst in which a heteropolyacid is supported on fumed silica obtained by a combustion method is disclosed as a supported catalyst for the production of ethanol by a hydration reaction of ethylene having improved performance (Patent Literature 4). As a method for improving the performance of a heteropolyacid supported catalyst, the use of a catalyst in which a heteropolyacid is supported on a clay carrier treated with a thermal acid has been proposed (Patent Literature 5).

As a carrier of a supported catalyst suitable for a hydration reaction of an olefin, a silica carrier in which a pore volume, a specific surface area, and a pore diameter are specified is disclosed, and a catalyst for producing ethanol by a hydration reaction of ethylene using the silica carrier is also exemplified (Patent Literature 6).

[CITATION LIST]

[PATENT LITERATURE]

[PTL 1] JP H03-80136 B
[PTL 2] JP 3041414 B
[PTL 3] JP 2001-79395 A
[PTL 4] JP 3901233 B

[PTL 5] JP H08-225473 A
[PTL 6] JP 2003-190786 A

SUMMARY OF INVENTION

[TECHNICAL PROBLEM]

Although an attempt has been made to improve the performance of a heteropolyacid supported catalyst in this way, it is desirable that a heteropolyacid supported catalyst can be stably used for a long period of time from an economic viewpoint, since the price of a heteropolyacid is expensive as compared with phosphoric acid. In particular, in the case that impurities are contained in a raw material, when a catalyst is used for a long period of time, the impurities or compounds to which the impurities are converted may accumulate on the catalyst surface, which would adversely affect the stable use of the catalyst. However, in general, the type of impurities affecting a catalytic reaction varies depending on the types of reaction and catalyst, and until now, it has not been clarified what impurities have an adverse effect on a hydration reaction of an olefin using a heteropolyacid catalyst.
It is an object of the present invention to provide a method for stably producing an alcohol having high product purity for a long period of time by a hydration reaction of an olefin using a heteropolyacid catalyst.

[SOLUTION TO PROBLEM]

As a result of intensive studies, the present inventors have found that impurity olefins contained in the main raw material cause the formation of a by-produced alcohol and an aldehyde compound, in the production of an alcohol by a hydration reaction of an olefin using a heteropolyacid catalyst. Accordingly, it has been confirmed that an alcohol having high product purity can be obtained by using a raw material having a small content of impurity olefins in a hydration reaction of an olefin using a heteropolyacid catalyst, and thus the present invention has been completed.
That is, the present invention relates to the following [1] to [9].

[1] A method for producing an alcohol comprising supplying a raw material mixture comprising water and an olefin having a carbon atom number of X, wherein X is an integer of 2 to 5, to a reactor, and subjecting them to a hydration reaction in a gas phase using a solid acid catalyst on which a heteropolyacid or a salt thereof is supported to obtain a reaction product, wherein the content of an impurity olefin having a carbon atom number of Y, wherein Y is an integer of 2 to 6 and Y and X are different, contained in the raw material mixture is 700 mol ppm or less.
[2] The method for producing an alcohol according to [1], wherein the raw material mixture comprises a recycled raw material obtained by separating the alcohol as a reaction target from the reaction product.
[3] The method for producing an alcohol according to [2], wherein the recycled raw material comprises at least one selected from the group consisting of unreacted water, an unreacted olefin having a carbon atom number of X, wherein X is an integer of 2 to 5, and an ether compound by-produced by the reaction.
[4] The method for producing an alcohol according to [2] or [3], comprising a step of separating the impurity olefin having a carbon atom number of Y contained in the recycled raw material from the raw material mixture or the recycled raw material.
[5] The method for producing an alcohol according to [4], wherein at least one selected from the group consisting of gas absorption, adsorption, distillation, and reaction conversion is used as a means for removing the impurity olefin having a carbon atom number of Y from the recycled raw material.
[6] The method for producing an alcohol according to any one of [1] to [5], wherein silica is used as a carrier of the solid acid catalyst.
[7] The method for producing an alcohol according to any one of [1] to [6], wherein the heteropolyacid is at least one compound selected from the group consisting of silicotungstic acid, phosphotungstic acid, phosphomolybdic acid, silicomolybdic acid, silicovanadotungstic acid, phosphovanadotungstic acid, and phosphovanadomolybdic acid.
[8] The method for producing an alcohol according to any one of [1] to [7], wherein the olefin having a carbon atom number of X is ethylene, wherein X is equal to 2, and the alcohol produced by the hydration reaction is ethanol.
[9] The method for producing an alcohol according to any one of [1] to [8], wherein the olefin having a carbon atom number of X is ethylene, wherein X is equal to 2, and the impurity olefin having a carbon atom number of Y is propylene or butene, wherein Y is equal to 3 or 4.

[ADVANTAGEOUS EFFECTS OF INVENTION]

According to the present invention, in the hydration reaction of an olefin using a heteropolyacid catalyst, by

supplying a raw material mixture having a small content of an impurity olefin to a reactor, generating of an alcohol and an aldehyde compound to be by-produced can be suppressed. As a result, it is possible to obtain an alcohol having high product purity and to reduce the cost required for a purification process.

BRIEF DESCRIPTION OF DRAWINGS

[Fig. 1] A diagram showing an example of an alcohol production process to which the present invention can be applied.

DESCRIPTION OF EMBODIMENTS

Hereinafter, preferred embodiments of the present invention will be described, but it should be understood that the present invention is not limited to these embodiments only, and various applications can be made within the spirit and practice of the present invention.

<Heteropolyacid catalyst>

A heteropolyacid catalyst according to one embodiment refers to a catalyst comprising a heteropolyacid or a salt thereof as a major active component of the catalyst.

<Heteropolyacid or salt thereof>

A heteropolyacid is composed of a central element and a peripheral element to which oxygen is bonded. The central element is usually silicon or phosphorus, but may be any one selected from a wide variety of elements of Groups 1 to 17 of the Periodic Table of the Elements.

Specific examples of the central element include a cupric ion; divalent ions of beryllium, zinc, cobalt and nickel; trivalent ions of boron, aluminum, gallium, iron, cerium, arsenic, antimony, phosphorus, bismuth, chromium and rhodium; tetravalent ions of silicon, germanium, tin, titanium, zirconium, vanadium, sulfur, tellurium, manganese, nickel, platinum, thorium, hafnium, cerium and other tetravalent rare earth ions; pentavalent ions of phosphorus, arsenic, vanadium, and antimony; a hexavalent ion of tellurium; and a heptavalent ion of iodine, but are not limited thereto.

Specific examples of the peripheral element include tungsten, molybdenum, vanadium, niobium, and tantalum, but are not limited thereto.

Such heteropolyacids are also known as "polyoxoanions", "polyoxometalates" or "metal oxide clusters". The structures of some of the wellknown anions are named after the researchers in this field, and for example, the Keggin structure, the Wells-Dawson structure and the Anderson-Evans-Perl off structure are known. Details are described in "Chemistry of Polyacids" (edited by the Chemical Society of Japan, Quarterly Chemical Review No. 20, 1993). A heteropolyacid usually has a high molecular weight, e.g., a molecular weight in the range of 700 to 8,500, and includes not only a monomer thereof but also a dimeric complex thereof.

The salt of the heteropolyacid is a metal salt or an onium salt in which some or all of the hydrogen atoms of the aforementioned heteropolyacid are substituted. Specific examples of the metal salt include the salt of lithium, sodium, potassium, cesium, magnesium, barium, copper, gold and gallium, and the specific examples of the onium salt include the salt of ammonia, etc., but are not limited thereto.

A heteropolyacid has relatively high solubility in polar solvents, such as water or other oxygenated solvents, particularly when the heteropolyacid is in the form of a free acid or some types of salts. The solubility of the heteropolyacid can be controlled by selecting an appropriate counterion.

Examples of the heteropolyacid which can be used in the catalyst include, but are not limited thereto, the following:

silicotungstic acid: $H_4[SiW_{12}O_{40}]\cdot xH_2O$
phosphotungstic acid: $H_3[PW_{12}O_{40}]\cdot xH_2O$
phosphomolybdic acid: $H_3[PMo_{12}O_{40}]\cdot xH_2O$
silicomolybdic acid: $H_4[SiMo_{12}O_{40}]\cdot xH_2O$
silicovanadotungstic acid: $H_{4+n}[SiV_nW_{12-n}O_{40}]\cdot xH_2O$
phosphovanadotungstic acid: $H_{3+n}[PV_nW_{12-n}O_{40}]\cdot xH_2O$
phosphovanadomolybdic acid: $H_{3+n}[PV_nMo_{12-n}O_{40}]\cdot xH_2O$
silicovanadomolybdic acid: $H_{4+n}[SiV_nMo_{12-n}O_{40}]\cdot xH_2O$
silicomolybdotungstic acid: $H_4[SiMo_nW_{12-n}O_{40}]\cdot xH_2O$
phosphomolybdotungstic acid: $H_3[PMo_nW_{12-n}O_{40}]\cdot xH_2O$

wherein n is an integer of 1 to 11 and x is an integer greater than or equal to 1.

The heteropolyacid is preferably silicotungstic acid, phosphotungstic acid, phosphomolybdic acid, silicomolybdic acid, silicovanadotungstic acid, phosphovanadotungstic acid, or phosphovanadomolybdic acid, and more preferably silicotungstic acid, phosphotungstic acid, silicovanadotungstic acid, or phosphovanadotungstic acid.

There is no particular limitation on the method for synthesizing such a heteropolyacid, and any methods may be used. For example, a heteropolyacid can be obtained by heating an acidic aqueous solution (approximately pH1 to pH2) containing a salt of molybdic acid or tungstic acid and a simple oxoacid of a heteroatom or a salt thereof. A heteropolyacid compound can be isolated, for example, by crystallization separation as a metal salt from the produced aqueous heteropolyacid solution.

Specific examples of the manufacture of the heteropolyacid are described on page 1413 of "New Experimental Chemistry 8, Synthesis of Inorganic Compound (III)" (edited by the Chemical Society of Japan, published by Maruzen Co., Ltd., August 20, 1984, third edition), but are not limited thereto. The structural confirmation of the synthesized heteropolyacid can be carried out by chemical analysis, as well as X-ray diffraction, UV, or IR measurements.

Preferred examples of the salt of the heteropolyacid include lithium salts, sodium salts, potassium salts, cesium salts, magnesium salts, barium salts, copper salts, gold salts, gallium salts, and ammonium salts of the aforementioned preferred heteropolyacids.

Specific examples of the salt of the heteropolyacid include a lithium salt of silicotungstic acid, a sodium salt of silicotungstic acid, a cesium salt of silicotungstic acid, a copper salt of silicotungstic acid, a gold salt of silicotungstic acid, a gallium salt of silicotungstic acid; a lithium salt of phosphotungstic acid, a sodium salt of phosphotungstic acid, a cesium salt of phosphotungstic acid, a copper salt of phosphotungstic acid, a gold salt of phosphotungstic acid, a gallium salt of phosphotungstic acid; a lithium salt of phosphomolybdic acid, a sodium salt of phosphomolybdic acid, a cesium salt of phosphomolybdic acid, a copper salt of phosphomolybdic acid, a gold salt of phosphomolybdic acid, a gallium salt of phosphomolybdic acid; a lithium salt of silicomolybdic acid, a sodium salt of silicomolybdic acid, a cesium salt of silicomolybdic acid, a copper salt of silicomolybdic acid, a gold salt of silicomolybdic acid, a gallium salt of silicomolybdic acid; a lithium salt of silicovanadotungstic acid, a sodium salt of silicovanadotungstic acid, a cesium salt of silicovanadotungstic acid, a copper salt of silicovanadotungstic acid, a gold salt of silicovanadotungstic acid, a gallium salt of silicovanadotungstic acid; a lithium salt of phosphovanadotungstic acid, a sodium salt of phosphovanadotungstic acid, a cesium salt of phosphovanadotungstic acid, a copper salt of phosphovanadotungstic acid, a gold salt of phosphovanadotungstic acid, a gallium salt of phosphovanadotungstic acid; a lithium salt of phosphovanadomolybdic acid, a sodium salt of phosphovanadomolybdic acid, a cesium salt of phosphovanadomolybdic acid, a copper salt of phosphovanadomolybdic acid, a gold salt of phosphovanadomolybdic acid, a gallium salt of phosphovanadomolybdic acid; a lithium salt of silicovanadomolybdic acid, a sodium salt of silicovanadomolybdic acid, a cesium salt of silicovanadomolybdic acid, a copper salt of silicovanadomolybdic acid, a gold salt of silicovanadomolybdic acid, and a gallium salt of silicovanadomolybdic acid.

The salt of the heteropolyacid is preferably a lithium salt of silicotungstic acid, a sodium salt of silicotungstic acid, a cesium salt of silicotungstic acid, a copper salt of silicotungstic acid, a gold salt of silicotungstic acid, a gallium salt of silicotungstic acid; a lithium salt of phosphotungstic acid, a sodium salt of phosphotungstic acid, a cesium salt of phosphotungstic acid, a copper salt of phosphotungstic acid, a gold salt of phosphotungstic acid, a gallium salt of phosphotungstic acid; a lithium salt of silicovanadotungstic acid, a sodium salt of silicovanadotungstic acid, a cesium salt of silicovanadotungstic acid, a copper salt of silicovanadotungstic acid, a gold salt of silicovanadotungstic acid, a gallium salt of silicovanadotungstic acid; a lithium salt of phosphovanadotungstic acid, a sodium salt of phosphovanadotungstic acid, a cesium salt of phosphovanadotungstic acid, a copper salt of phosphovanadotungstic acid, a gold salt of phosphovanadotungstic acid, or a gallium salt of phosphovanadotungstic acid.

As the salt of the heteropolyacid, it is particularly preferable that a lithium salt of silicotungstic acid, a cesium salt of silicotungstic acid, a lithium salt of phosphotungstic acid or a cesium salt of phosphotungstic acid be used.

<Carrier>

The heteropolyacid catalyst can be used as it is, but is preferably used as supported on a carrier. The carrier is preferably at least one selected from the group consisting of silica, diatomaceous earth, titania, activated carbon, alumina, and silica alumina, and more preferably silica.

The shape of the carrier is not particularly limited. Examples of the shape include a spherical shape, a cylindrical shape, a hollow cylindrical shape, a plate shape, an elliptical shape, a sheet shape, and a honeycomb shape. The shape is preferably spherical, cylindrical, hollow cylindrical, or elliptical, and more preferably spherical or cylindrical, in order to facilitate filling into the reactor and supporting of a catalytically active component.

Although the size of the carrier is not particularly limited, it is desirable that the size be determined by taking into consideration handling at the time of producing a solid acid catalyst on which a catalytically active component is supported or at the time of filling the catalyst, the differential pressure after filling it into a reactor, the reaction performance

of the catalytic reaction, etc., since they are affected by the size of the carrier. The size of the carrier is preferably 1 mm to 20 mm, and more preferably 2 mm to 10 mm, when used in a fixed bed system.

Although there is no limitation on the strength of the carrier, the crush strength of the carrier is preferably 5 N or more, and more preferably 10 N or more, since cracking or breakage of a solid acid catalyst causes an increase in the differential pressure of a reactor or occlusion of a pipe. In the present disclosure, the crush strength is a value obtained when a load is applied to a carrier by using a digital hardness meter KHT-40N type manufactured by FUJIWARA SCIENTIFIC CO., LTD., and the carrier is crushed.

Although there is no limitation on the specific surface area of the carrier, the specific surface area by the BET method is preferably 50 $m^2/g$ or more, and more preferably 100 $m^2/g$ or more, since the activity of the catalyst increases as the specific surface area increases.

There is no particular limitation on the method for supporting the heteropolyacid or the salt thereof on the carrier. In general, it can be carried out by making the carrier absorb a solution or suspension obtained by dissolving or suspending the heteropolyacid or the salt thereof in a solvent and evaporating the solvent.

The amount of the heteropolyacid or the salt thereof to be supported on the carrier can be adjusted, for example, by dissolving the heteropolyacid or the salt thereof in an amount of distilled water that corresponds to the amount of water absorbed by the carrier, and impregnating the carrier with the solution. In another embodiment, the amount of the heteropolyacid or the salt thereof to be supported on the carrier can also be adjusted by immersing the carrier in a solution of an excess amount of the heteropolyacid or the salt thereof with moderate movement, followed by filtration to remove an excess heteropolyacid or salt thereof.

The volume of the solution or suspension varies depending on the carrier used, the supporting method, etc. By placing a carrier in which the heteropolyacid or the salt thereof is impregnated in a heating oven for several hours to evaporate a solvent, a solid acid catalyst supported on the carrier can be obtained. The drying method is not particularly limited, and various methods, such as a stationary method, and a belt conveyor method, can be used. The amount of the heteropolyacid or the salt thereof supported on the carrier can be accurately measured by chemical analysis, such as ICP and XRF.

The amount of the heteropolyacid or the salt thereof supported on the carrier is preferably 10 to 300 parts by mass, and more preferably 20 to 200 parts by mass, in terms of the total mass of the heteropolyacid and the salt thereof with respect to 100 parts by mass of the carrier.

<Method for producing alcohol by hydration reaction of olefin>

Next, a method for producing an alcohol by a hydration reaction of an olefin using a heteropolyacid catalyst will be described. An alcohol can be obtained by supplying a raw material mixture comprising water and an olefin having a carbon atom number of X, wherein X is an integer of 2 to 5, to a reactor, and subjecting them to a hydration reaction in a gas phase using a solid acid catalyst on which the heteropolyacid or the salt thereof is supported to obtain a reaction product.

A specific example of the alcohol production reaction by the hydration reaction of an olefin having a carbon atom number of X, wherein X is an integer of 2 to 5 is represented by Formula (1):

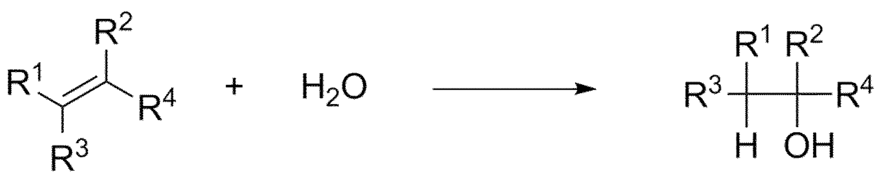
(1)

wherein $R^1$ to $R^4$ each independently represent a hydrogen atom or an alkyl group having 1 to 3 carbon atoms, and the sum of the carbon atoms of $R^1$ to $R^4$ is 0 to 3.

There is no particular limitation on the olefin having a carbon atom number of X, wherein X is an integer of 2 to 5 which can be used in the hydration reaction of an olefin using the heteropolyacid catalyst. The olefin having a carbon atom number of X, wherein X is an integer of 2 to 5, is preferably ethylene; propylene; butene, specifically, 1-butene, 2-butene, and isobutene; pentene; or a mixture of two or more thereof. Among them, ethylene is more preferable.

Although there is no limitation on the use ratio of the olefin and water, the molar ratio of the olefin to water is preferably water/olefin = 0.01 to 2.0, and more preferably water/olefin = 0.1 to 1.0, since the concentration dependence of the olefin on the reaction rate is large and the energy cost of the alcohol production process increases when the water concentration is high.

There is no limitation on the mode of the hydration reaction of an olefin using the heteropolyacid catalyst, and any of the reaction modes can be used. From the viewpoint of ease of separation from the catalyst and reaction efficiency,

the preferred examples of the mode include a fixed-bed, a fluidized-bed, and a suspension-bed. A fixed-bed that requires the least energy for separation from the catalyst is more preferable.

The gas space velocity in the reactor in the case of using a fixed-bed is not particularly limited, but is preferably 500 to 15,000/hr, and more preferably 1000 to 10,000/hr, from the viewpoint of energy and reaction efficiency. When the gas space velocity is 500/hr or more, the amount of the catalyst used can be effectively reduced, and when the gas space velocity is 15000/hr or less, the amount of gas circulation can be reduced, so that the production of alcohol can be more efficiently carried out within the above ranges.

There is no limitation on the reaction pressure in the hydration reaction of an olefin using the heteropolyacid catalyst. Since the hydration reaction of an olefin is a reaction in which the number of molecules decreases, it is generally advantageous to proceed at high pressure. The reaction pressure is preferably 0.5 to 7.0 MPaG, and more preferably 1.5 to 4.0 MPaG. "G" means a gauge pressure. When the reaction pressure is 0.5 MPaG or more, a satisfactory reaction rate can be obtained, and when the reaction pressure is 7.0 MPaG or less, it is possible to eliminate the necessity of installation of equipment as countermeasures for condensation of an olefin and in relation to evaporation of an olefin, and equipment for high pressure gas safety, and moreover, further reduce costs regarding energy.

The reaction temperature of the hydration reaction of an olefin using the heteropolyacid catalyst is not particularly limited, and the reaction can be carried out at a wide range of temperatures. In view of the thermal stability of the heteropolyacid or the salt thereof and the temperature at which water, one of the raw materials, does not condense, the preferred reaction temperature is 100 to 550°C, and more preferably 150 to 350°C.

The hydration reaction of an olefin using the heteropolyacid catalyst is an equilibrium reaction, and the conversion rate of olefin will be an equilibrium conversion rate at most. For example, the equilibrium conversion rate in the production of ethanol by the hydration of ethylene is calculated to be 7.5% at a temperature of 200°C and a pressure of 2.0 MPaG. Therefore, in the method for producing an alcohol by the hydration of an olefin, a maximum conversion rate is determined by the equilibrium conversion rate, and as can be seen in an example of ethylene, the hydration reaction of an olefin tends to have a small equilibrium conversion rate, and thus it is strongly required in the industry to carry out the hydration reaction of an olefin with high efficiency under mild conditions.

In the hydration reaction of an olefin using the heteropolyacid catalyst, loss of the olefin can be reduced by recycling any unreacted olefin having a carbon atom number of X, wherein X is an integer of 2 to 5 into a reactor. There is no limitation on the method for recycling the unreacted olefin into the reactor, and from the reaction product which is a process fluid coming out of the reactor, the alcohol as the reaction target product may be separated, and the recycled raw material comprising the unreacted olefin may be recycled into the reactor, or may be recycled together with other inert components.

Typically, an industrial grade olefin often contains a very small amount of paraffin. Therefore, for example, when ethylene containing ethane is used and the unreacted ethylene is recycled to the reactor, it is desirable to purge a portion of the reaction gas, which is ethylene gas, recovered from the reaction product to the outside of the system, in order to prevent concentration and accumulation of ethane.

In the hydration reaction of an olefin using the heteropolyacid catalyst, the produced alcohol may be dehydrated to generate an ether compound as a by-product. For example, when ethanol is obtained by the hydration of ethylene, diethyl ether is by-produced. It is considered that this diethyl ether is generated by a dehydration reaction of two molecules of ethanol, and when ethanol is produced by the hydration reaction of ethylene, the reaction yield is remarkably lowered.

However, by recycling the by-produced diethyl ether into the reactor, diethyl ether is converted into ethanol, so that ethanol can be produced from ethylene with extremely high efficiency. Although there is no particular limitation on the method for recycling the by-produced ether compound into the reactor, there are, for example, a method including isolating an ether compound from components distilled from the reactor and recycling the ether compound into the reactor, and a method including recycling the ether compound into the reactor as a gas component together with an unreacted olefin.

In the hydration reaction of an olefin using the heteropolyacid catalyst, the produced alcohol in a state of being dissolved in a large amount of water which has not been converted as a reaction raw material is sent to a separation and purification step together with other by-products. In the separation and purification step, the alcohol, water, and the other by-products are separated, and an alcohol having a purity equal to or higher than a certain level by the purification is obtained as a product.

At this time, the water simultaneously obtained may be disposed of as waste water, but from the viewpoint of impact or load on the environment, it is desirable to recycle it in the process and use it again as a raw material for the reaction. There is no limitation on the type and number of apparatuses in the separation and purification step, and a distillation apparatus or a membrane separation apparatus may be used, and different apparatuses can be used in combination if necessary.

An example of a production process using a recycled raw material is shown in FIG. 1. The present invention is not limited by the flow of FIG. 1. In the process flow of FIG. 1, the recycled raw material comprising an unreacted recycled olefin gas 4, which is a gas of an olefin having a carbon atom number of X, unreacted recycled water 8, and

by-produced recycled ether 6, together with a raw material olefin gas 10 and raw material water 11 are supplied to an evaporator 1, mixed and gasified, and supplied to a reactor 2 as a raw material mixture 12.

A reaction product 13 is sent to an intermediate tank 3, and an unreacted olefin gas is separated from the reaction product 13 in the intermediate tank 3, and is supplied to the evaporator 1 as a recycled olefin gas 4. It is preferable that a portion of the recycled olefin gas 4 be discharged (purged) out of the system as described above.

In a low boiling point component removal tower 5, a by-produced ether compound which is a low boiling point component is separated from the top of the tower by distillation or the like, and is returned to the evaporator 1 as a recycled ether 6, which is then included in the reaction raw material. A high boiling point component drawn out from the bottom of the low boiling point component removal tower 5 is distilled and separated into a crude alcohol 9 which is a low boiling point component as a reaction target component and water which is a high boiling point component in a water removal tower 7. Water drawn out from the bottom of the water removal tower 7 is returned to the evaporator 1 as recycled water 8. If necessary, the crude alcohol 9 can be further purified to obtain a product alcohol.

In the hydration reaction of an olefin using the heteropolyacid catalyst, it is possible to reuse an unreacted olefin, a by-produced ether compound, and water as a recycled raw material, and also to use a by-product of another production process as a raw material, as described above. There is no limitation on the source of these raw materials.

In studying the use of a plurality of raw materials, the present inventors have found that impurities mixed in a raw material affect the reaction and the state of a catalyst depending on the types of the impurities. In particular, an aldehyde compound has high polymerization reactivity and polymerizes on a solid acid catalyst surface to form a coke, which reduces the reaction activity of the catalyst or deteriorates the selectivity thereof.

The aldehyde compound is supplied to a reactor as an impurity in a raw material and a reaction by-product contained in a recycled raw material. Examples of the aldehyde compound include acetaldehyde, butyraldehyde, crotonaldehyde, and hexanal.

It is presumed that the aldehyde compound of the reaction by-product is by-produced by dehydrogenation of an alcohol compound. For example, it is considered that acetaldehyde is by-produced from ethanol and butyraldehyde is by-produced from butanol.

In order to suppress the formation of an aldehyde compound, it has been found that an impurity olefin having a carbon atom number of Y, wherein Y is an integer of 2 to 6 and Y and X are different, contained in a raw material supplied to a reactor may be set to a certain amount or less. Further, when the amount of impurity olefin having a carbon atom number of Y is small, it is possible to reduce the amount of by-produced alcohol as well, and it is possible to increase the purity of the target alcohol.

An impurity olefin is an olefin different from a reaction raw material olefin having a carbon atom number of X, wherein X is an integer of 2 to 5. That is, the carbon atom number of Y of the impurity olefin is an integer of 2 to 6, and Y and X are different.

Examples of the impurity olefin having a carbon atom number of Y include ethylene; propylene; butene, specifically, 1-butene, 2-butene, isobutene; pentene; and hexene. When an olefin having a carbon atom number of X, wherein X is an integer of 2 to 5, as a reaction raw material is ethylene, wherein X = 2, the carbon atom number of Y of the impurity olefin is often 3 or 4. In this case, the impurity olefin is propylene or butene, and is often at least one selected from the group consisting of n-butene which is at least one of 1-butene or 2-butene, and isobutene.

The total concentration, hereinafter, referred to as reactor inlet concentration, of the impurity olefin having a carbon atom number of Y, wherein Y is an integer of 2 to 6 and Y and X are different, contained in the raw material mixture supplied to the reactor is 700 mol ppm or less, and more preferably 500 mol ppm or less. The lower limit of the reactor inlet concentration of the impurity olefin is not particularly limited, but may be, for example, 0.1 mol ppm, or 1 mol ppm.

There is no limitation on the means for lowering the reactor inlet concentration of the impurity olefin. For example, by separating a raw material, which may also include a recycled raw material, and an impurity olefin using a separation and purification apparatus, the impurity olefin may be separated or removed from the raw material or the recycled raw material, and a raw material or the like having a reduced impurity olefin concentration may be returned to a supply, or a plurality of raw materials may be appropriately combined so that the impurity olefin concentration does not exceed a certain value. Alternatively, the concentration of the impurity olefin at the inlet of the reactor may be lowered by raising the pressure of the recycle raw material again by a compressor or the like, then purging a part of the recycle raw material to the outside of the system, and supplying the remainder to the reactor.

Although there is no limitation on the means for separating the raw material and the impurity olefin or removing the impurity olefin from the raw material, at least one selected from the group consisting of gas absorption, adsorption, distillation, and reaction conversion can be used. For the separation of the raw material and the impurity olefin, a distillation tower, an absorption tower, an adsorption apparatus, a membrane separation apparatus or the like may be used. Since the raw material mixture contains a plurality of types of raw materials, such as an olefin, an ether compound, and water, a different treatment may be applied to each individual raw material. For example, in the absorption tower, the impurity olefin can be selectively absorbed and removed by using a suitable organic solvent. In other words, an efficient separation

means can be appropriately selected based on the type and properties of the impurity olefin and the type and properties of the raw material.

EXAMPLES

Although the present invention will be further described with reference to the following Examples and Comparative Examples, these examples illustrate the summary of the present invention, and the present invention is not limited to these examples.

1. Preparation of silica carrier A

25 parts by mass of fumed silica: Aerosil (trademark) 300 manufactured by Nippon Aerosil Co., Ltd., 75 parts by mass of silica gel: CARiACT G6 manufactured by Fuji Silysia Chemical, Ltd., and 45 parts by mass of colloidal silica: SNOWTEX (trademark) O manufactured by Nissan Chemical Corporation (9 parts by mass in terms of solid content) were kneaded by a kneader, and then water and 10 parts by mass of methyl cellulose: METOLOSE (trademark) SM-4000 manufactured by Shin-Etsu Chemical Co., Ltd. as an additive, and 5 parts by mass of a resin-based binder: Cerander (trademark) YB-132A manufactured by Yuken Industry Co., Ltd. were added, with the status of a mixture being monitored, and the mixture was further kneaded to obtain a kneaded material.

Next, the kneaded material was put into an extruder, to which a die having a circular hole of 6 mmφ was attached. The kneaded material was extruded from the extruder, and the extruded intermediate material was cutwith a cutter so that the extruded intermediate material had the same length as the diameter of the circular hole (6 mm) used to obtain a cylindrical shaped body before calcination. The obtained shaped body before calcination was formed into a spherical shape by Marumerizer (trademark), and then dried at 70°C for 24 hours or more, and calcined at about 820°C under an air atmosphere, and cooled to obtain a silica carrier A.

(Measurement of water absorption rate of silica sarrier A)

The water absorption rate of the obtained silica carrier A was measured by the following method.

(1) Approximately 5 g of the carrier was weighed (W1 (g)) on a balance and placed in a 100 mL beaker.
(2) Approximately 15 mL of pure water (ion-exchanged water) was added to the beaker so as to completely cover the carrier.
(3) Left for 30 minutes.
(4) The carrier and pure water were flowed over a wire net, and pure water was removed by the wire net, and the carrier was taken out.
(5) Water adhering to the surface of the carrier was removed by lightly pressing with a paper towel until there was no gloss on the surface.
(6) The total mass of the carrier and pure water obtained in (5) was measured (W2 (g)).
(7) The water absorption rate of the carrier was calculated by the following formula.

$$\text{Water absorption (g - water / g - carrier) (\%)} = (W2 - W1) / W1 \times 100$$

Therefore, the water absorption amount of the carrier (g) can be calculated by "the water absorption rate of the carrier (g - water / g - carrier) (%) × the mass of the used carrier (g) ".

2. Preparation of solid acid catalyst A in which heteropolyacid is supported on silica carrier A

In a 100 mL beaker, 40.7 g of a commercially available Keggin silicotungstic acid 26 hydrate ($H_4SiW_{12}O_{40}\cdot26H_2O$; manufactured by Nippon Inorganic Colour & Chemical Co., Ltd.) was weighed, a small amount of distilled water was added to solve silicotungstic acid, and then the solution was transferred to a 200 mL graduated cylinder. Then, distilled water was added so that the liquid amount of the silicotungstic acid solution in the graduated cylinder was 95% of the water absorption rate of the silica carrier A to be used, and the mixture was stirred so that the entire mixture was uniform. After the stirring, the aqueous solution of silicotungstic acid was transferred to a 200 mL volumetric flask, and then weighed 100 mL of the silica carrier A was put into the 200 mL volumetric flask, and the contents of the volumetric flask were mixed so that the aqueous solution of silicotungstic acid contacted the entire carrier and silicotungstic acid was supported on the silica carrier A. The silica carrier A on which silicotungstic acid was supported was transferred to a porcelain dish, air-dried for one hour, and then dried for 5 hours in a hot air dryer adjusted to 150°C.

After the drying, the silica carrier A was transferred into a desiccator and cooled to room temperature to obtain a solid acid catalyst A.

3. Hydration reaction of ethylene

A reactor filled with a predetermined amount of the solid acid catalyst A was controlled to reach a predetermined temperature and to be pressurized to a predetermined pressure, and a predetermined amount of water vaporized by an evaporator and a predetermined amount of ethylene from a mass flow controller were introduced into the reactor.

(Calculation of reaction results)

A reaction gas after passing through the reactor was cooled, and a condensed liquid (reaction solution) and the reaction gas from which the condensate (reaction solution) was removed were sampled for a certain period of time, respectively. The sampled liquid (reaction solution) and the reaction gas were analyzed by the following methods using a gas chromatography analyzer and a Karl Fischer analyzer to calculate the reaction results.

4. Analysis of reaction gas

The sampled reaction gas was analyzed by using a gas chromatography apparatus (apparatus name: 7890) manufactured by Agilent Technologies Japan, Ltd., and a system program based on a plurality of columns and two detectors.

- Gas chromatography conditions:

  Oven: kept at 40°C for 3 minutes, then raised to 200°C at 20°C/min
  Carrier gas: helium
  Split ratio: 10:1

- Columns used: manufactured by Agilent Technologies Japan, Ltd.

  HP-1: 2 m
  GasPro: 30 m × 320 μm
  DB-624: 60 m × 320 μm × 1.8 μm

- Detectors:

  Front detector: FID (heater: 230°C, hydrogen flow rate 40 mL/min, air flow rate 400 L/min)
  Back detector: FID (heater: 230°C, hydrogen flow rate 40 mL/min, air flow rate 400 L/min)
  Aux detector: TCD (heater: 230°C, reference flow rate 45 mL/min, make-up flow rate 2 mL/min)

5. Analysis of reaction solution

The sampled reaction solution was analyzed by using a gas chromatography apparatus (apparatus name: 6850) manufactured by Agilent Technologies Japan, Ltd. Further, the concentration of water in the reaction solution was analyzed by a Karl Fischer analyzer manufactured by Mitsubishi Chemical Co., Ltd.

- Columns used: PoraBOND Q 25 m × 0.53 mm ID × 10 μm
- Oven temperature: kept at 100°C for 2 minutes, then raised to 240°C at 5°C/min.
- Injection temperature: 250°C
- Detector temperature: 300°C

<Example 1>

(Hydration reaction of ethylene)

4 mL of the solid acid catalyst A was weighed, and filled in a tubular reactor (made from SUS316, inner diameter of 10 mm, length of 300 mm), which was then pressurized to 0.75 MPaG after nitrogen gas replacement. Then, the reactor was heated to 160°C, and at a stage where the temperature was stable, water vaporized by the evaporator and

ethylene were fed into the reactor under the conditions that GHSV (gas space velocity) was 2000/hr and the molar ratio of water to ethylene was 0.3 to carry out a hydration reaction of ethylene.

(Calculation of reaction results)

After feeding of water and ethylene, the temperature was adjusted so that the ethanol production STY (kg/$m^3$/h) was about 80. Thereafter, a gas passed through the reactor was cooled, and sampling of a condensed liquid (reaction solution) and a reaction gas from which the condensed liquid (reaction solution) was removed was each carried out for 1 hours, and the acquired condensed liquid (reaction solution) and the reaction gas were respectively analyzed by the above methods, and the reaction results were calculated from the masses of the condensed liquid (reaction solution) and the reaction gas, the gas flow rates, and the analysis results.

<Example 2>

The reaction was carried out in the same manner as in Example 1, instead of ethylene used in Example 1, using ethylene prepared so that the reactor inlet concentration of butene was 220 mol ppm as a raw material, and the reaction result was calculated.

<Example 3>

The reaction was carried out in the same manner as in Example 1, instead of ethylene used in Example 1, using ethylene prepared so that the reactor inlet concentration of butene was 430 mol ppm as a raw material, and the reaction result was calculated.

<Example 4>

The reaction was carried out in the same manner as in Example 1, instead of ethylene used in Example 1, using ethylene prepared so that the reactor inlet concentration of butene was 580 mol ppm as a raw material, and the reaction result was calculated.

<Comparative Example 1>

The reaction was carried out in the same manner as in Example 1, instead of ethylene used in Example 1, using ethylene prepared so that the reactor inlet concentration of butene was 720 mol ppm as a raw material, and the reaction result was calculated.

<Reaction results>

The reaction results of Examples 1 to 4 and Comparative Example 1 are shown in Table 1. Note that butene in the table is the total amount of 1-butene, 2-butene, and isobutene, and the selectivity ratio of butene was calculated by subtracting the amount of butene at the inlet of the reactor from the total amount of butene in the outlet gas analysis of the reaction, and using this value as a ratio to the amount of ethylene supplied.

In Example 1 without butene added, butene was produced from ethylene at an ethylene conversion of 6% and a selectivity of 0.71%. In Comparative Example 1, the reactor inlet concentration of butene was as high as 720 mol ppm, and the selectivity ratios of acetaldehyde, butane, and 2-butanol, which are by-products, were higher than those of Examples 1 to 4.

Further, from the results of Examples 1 to 4, it can be understood that, by reducing the reactor inlet concentration of butene, the selectivity ratios of acetaldehyde, butane, and 2-butanol, which are by-products, can be reduced, and a product with less impurities can be obtained. Further, it can be said that, by reducing the reactor inlet concentration of butene, the selectivity of acetaldehyde, which lowers the reaction activity of the catalyst or deteriorates the selectivity, is reduced, so that the alcohol can be stably produced for a long period of time.

Note that, when the butene concentration at the inlet of the reactor increases, the butene selectivity of ethylene apparently tends to decrease. This is considered to be due to the balance between the amount of butene by-produced from ethylene and the amount of butene converted (consumed) into 2-butanol, butane, and the like.

Table 1

Table 1

| | Reactor inlet concentration of butene [mol ppm] | Ethanol production STY [kg/m$^3$/h] | Selectivity ratios of by-products | | | |
|---|---|---|---|---|---|---|
| | | | acetaldehyde | butane | 2-butanol | butene |
| Example 1 | 0 | 79 | 0.40% | 0.10% | 0.00% | 0.71% |
| Example 2 | 220 | 81 | 0.50% | 0.10% | 0.00% | 0.34% |
| Example 3 | 430 | 79 | 0.80% | 0.17% | 0.00% | 0.07% |
| Example 4 | 580 | 80 | 1.30% | 0.41% | 0.02% | 0.01% |
| Comparative Example 1 | 720 | 79 | 1.40% | 0.45% | 0.02% | 0.01% |

<Example 5>

In this example, the gas passed through the reactor was separated into a cooled, and condensed liquid (reaction solution) and a reaction gas from which the condensed liquid (reaction solution) was removed, and then the reaction gas containing ethylene as a main component was subjected to a hydration reaction of ethylene by a method in which a part of the reaction gas was purged after re-pressurization in a compressor and the remainder was recycled into a reactor.

280 mL of the solid acid catalyst A was weighed, and filled in a tubular reactor (made from SUS316, inner diameter of 34 mm, length of 1500 mm), which was then pressurized to 2.4 MPaG after nitrogen gas replacement. Then, the reactor was heated to 180°C, and at a stage where the temperature was stable, water, ethylene, and a recycled gas were fed into the reactor under the conditions that GHSV (gas space velocity) was 3000/hr and the molar ratio of water to ethylene was 0.3 to carry out a hydration reaction of ethylene.

After feeding of water, ethylene, and the recycled gas, the temperature was adjusted so that the ethanol production STY (kg/m$^3$/h) was about 150. By adjusting the purge amount of the recycled gas, the concentration of butene accumulated in the recycled gas was adjusted so that the reactor inlet butene concentration was 350 mol ppm. Thereafter, a gas passed through the reactor was cooled, and sampling of a reaction solution and a reaction gas was each carried out for 1 hours, and the reaction results were calculated from the masses of the condensed liquid and the reaction gas, the gas flow rates, and the analysis results.

<Example 6>

A hydration reaction of ethylene was carried out in the same manner as in Example 5, except that the purge amount of the recycled gas was adjusted so that the butene concentration at the inlet of the reactor was 550 mol ppm, and the reaction result was calculated.

<Comparative Example 2>

A hydration reaction of ethylene was carried out in the same manner as in Example 5, except that the purge amount of the recycled gas was adjusted so that the butene concentration at the inlet of the reactor was 710 mol ppm, and the reaction result was calculated.

<Reaction results>

The reaction results of Examples 5 to 6 and Comparative Example 2 are shown in Table 2. In Comparative Example 2, the reactor inlet concentration of butene was as high as 720 mol ppm, and the selectivity ratios of acetaldehyde, butane, and 2-butanol, which are by-products, were higher than those of Examples 5 to 6.

Further, from the results of Examples 5 to 6, it can be understood that, by reducing the reactor inlet concentration of butene, the selectivity ratios of acetaldehyde, butane, and 2-butanol, which are by-products, can be reduced, and a product with less impurities can be obtained. Further, it can be said that, by reducing the reactor inlet concentration of butene, the selectivity of acetaldehyde, which lowers the reaction activity of the catalyst or deteriorates the selectivity, is reduced, so that the alcohol can be stably produced for a long period of time.

Table 2

Table 2

| | Reactor inlet concentration of butene [mol ppm] | Ethanol production STY [kg/m$^3$/h] | Selectivity ratios of by-products | | | |
|---|---|---|---|---|---|---|
| | | | acetaldehyde | butane | 2-butanol | butene |
| Example 5 | 350 | 149 | 0.045% | 0.0004% | 0.026% | 0.002% |
| Example 6 | 550 | 149 | 0.070% | 0.0018% | 0.037% | 0.006% |
| Comparative Example 2 | 710 | 150 | 0.090% | 0.0031% | 0.046% | 0.005% |

INDUSTRIAL APPLICABILITY

The present invention is industrially useful in that, in the production of an alcohol by a hydration reaction of an olefin using a heteropolyacid catalyst, an alcohol product with less by-products can be stably produced by reducing an impurity olefin concentration in a raw material, and a cost required for a purification process can be reduced.

REFERENCE SIGNS LIST

1: Evaporator
2: Reactor
3: Intermediate tank
4: Recycled olefin gas
5: Low boiling point component removal tower
6: Recycled ether
7: Water removal tower
8: Recycled water
9: Crude alcohol
10: Raw material olefin gas
11: Raw material water
12: Raw material mixture
13: Reaction product

**Claims**

1. A method for producing an alcohol comprising supplying a raw material mixture comprising water and an olefin having a carbon atom number of X, wherein X is an integer of 2 to 5, to a reactor, and subjecting them to a hydration reaction in a gas phase using a solid acid catalyst on which a heteropolyacid or a salt thereof is supported to obtain a reaction product, wherein the content of an impurity olefin having a carbon atom number of Y, wherein Y is an integer of 2 to 6 and Y and X are different, contained in the raw material mixture is 700 mol ppm or less.

2. The method for producing an alcohol according to claim 1, wherein the raw material mixture comprises a recycled raw material obtained by separating the alcohol as a reaction target from the reaction product.

3. The method for producing an alcohol according to claim 2, wherein the recycled raw material comprises at least one selected from the group consisting of unreacted water, an unreacted olefin having a carbon atom number of X, wherein X is an integer of 2 to 5, and an ether compound by-produced by the reaction.

4. The method for producing an alcohol according to claim 2 or 3, comprising a step of separating the impurity olefin having a carbon atom number of Y contained in the recycled raw material from the raw material mixture or the

recycled raw material.

5. The method for producing an alcohol according to claim 4, wherein at least one selected from the group consisting of gas absorption, adsorption, distillation, and reaction conversion is used as a means for removing the impurity olefin having a carbon atom number of Y from the recycled raw material.

6. The method for producing an alcohol according to any one of claims 1 to 5, wherein silica is used as a carrier of the solid acid catalyst.

7. The method for producing an alcohol according to any one of claims 1 to 6, wherein the heteropolyacid is at least one compound selected from the group consisting of silicotungstic acid, phosphotungstic acid, phosphomolybdic acid, silicomolybdic acid, silicovanadotungstic acid, phosphovanadotungstic acid, and phosphovanadomolybdic acid.

8. The method for producing an alcohol according to any one of claims 1 to 7, wherein the olefin having a carbon atom number of X is ethylene, wherein X is equal to 2, and the alcohol produced by the hydration reaction is ethanol.

9. The method for producing an alcohol according to any one of claims 1 to 8, wherein the olefin having a carbon atom number of X is ethylene, wherein X is equal to 2, and the impurity olefin having a carbon atom number of Y is propylene or butene, wherein Y is equal to 3 or 4.

FIG. 1

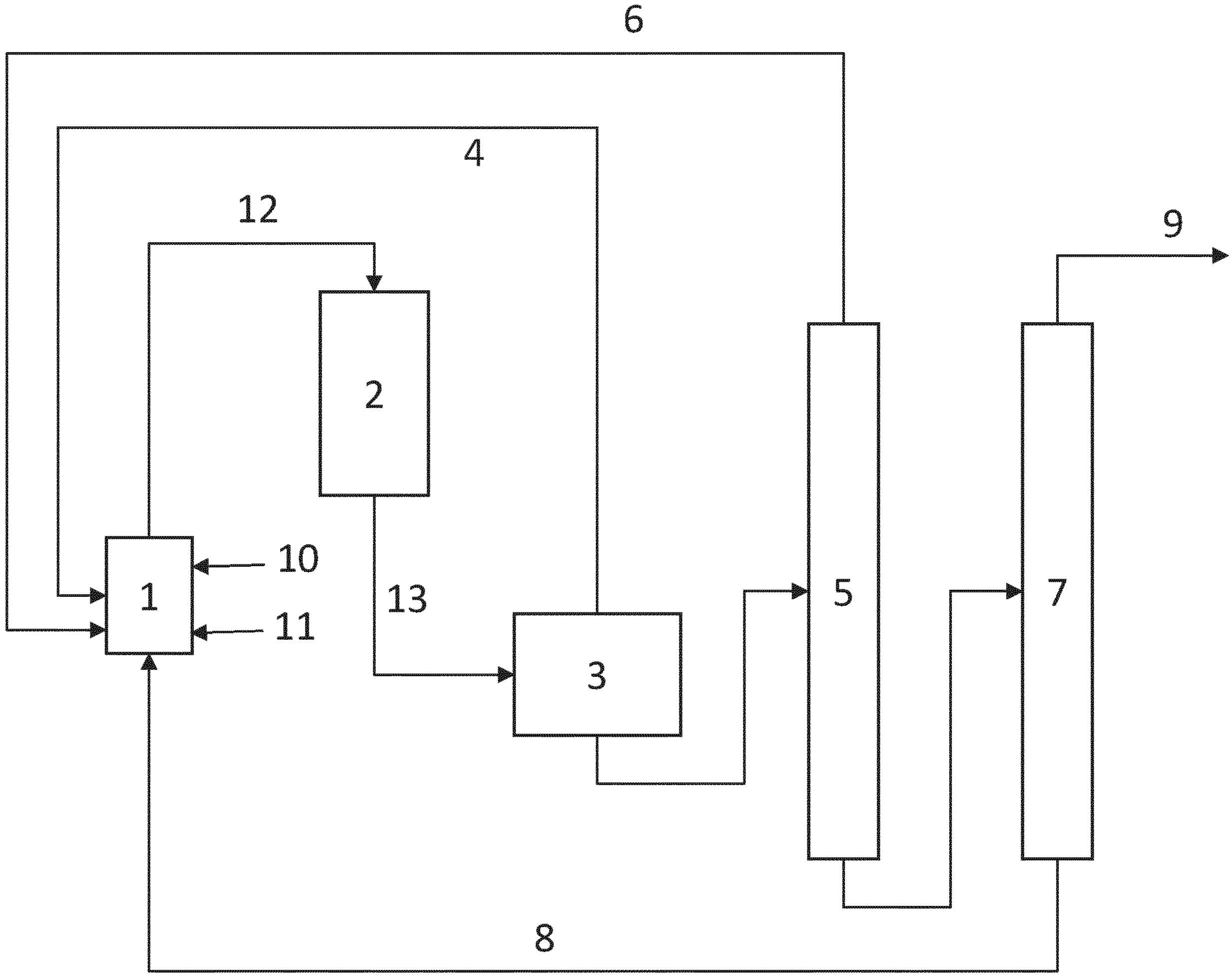

## INTERNATIONAL SEARCH REPORT

International application No.
PCT/JP2021/012703

A. CLASSIFICATION OF SUBJECT MATTER
Int. Cl. C07C29/04(2006.01)i, C07C31/08(2006.01)i, C07B61/00(2006.01)i, B01J23/30(2006.01)i
FI: C07C29/04, C07C31/08, B01J23/30 Z, C07B61/00 300
According to International Patent Classification (IPC) or to both national classification and IPC

B. FIELDS SEARCHED
Minimum documentation searched (classification system followed by classification symbols)
Int. Cl. C07C29/04, C07C31/08, C07B61/00, B01J23/30

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
Japio-GPG/FX

C. DOCUMENTS CONSIDERED TO BE RELEVANT

| Category* | Citation of document, with indication, where appropriate, of the relevant passages | Relevant to claim No. |
|---|---|---|
| X | JP 8-192047 A (BP CHEMICALS INTERNATIONAL LIMITED) 30 July 1996, claims, paragraph [0023], examples | 1-9 |
| X | JP 9-262475 A (TSUSHO SANGYOSHO KISO SANGYO KYOKUCHO) 07 October 1997, claims, paragraph [0015], examples | 1-9 |
| X<br>A | JP 9-263554 A (TSUSHO SANGYOSHO KISO SANGYO KYOKUCHO) 07 October 1997, claims, paragraph [0008], examples | 1-6, 8-9<br>7 |
| X<br>A | JP 11-300207 A (TSUSHO SANGYOSHO KISO SANGYO KYOKUCHO) 02 November 1999, claims, paragraph [0013], examples | 1-6, 8-9<br>7 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06.05.2021 | 18.05.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2021/012703

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2000-26343 A (BP CHEMICALS INTERNATIONAL LIMITED) 25 January 2000, claims, paragraph [0018], examples | 1-7<br>8-9 |
| A | JP 10-101601 A (NIPPON ETANOOLE KK) 21 April 1998, claims, examples | 1-9 |
| A | JP 2002-145813 A (NATIONAL INSTITUTE OF ADVANCED INDUSTRIAL SCIENCE AND TECHNOLOGY) 22 May 2002, claims, examples | 1-9 |
| A | WO 2007/105421 A1 (IDEMITSU KOSAN CO., LTD.) 20 September 2007, examples, claims | 1-9 |
| P, X | WO 2020/090756 A1 (SHOWA DENKO KABUSHIKI KAISHA) 07 May 2020, examples, claims | 1-9 |

Form PCT/ISA/210 (continuation of second sheet) (January 2015)

**INTERNATIONAL SEARCH REPORT**
Information on patent family members

International application No.
PCT/JP2021/012703

| Patent Documents referred to in the Report | Publication Date | Patent Family | Publication Date |
|---|---|---|---|
| JP 8-192047 A | 30.07.1996 | US 5684216 A<br>columns 3, 4,<br>examples, claims<br>EP 704240 A1<br>CN 1130103 A<br>KR 10-1996-0010074 A | |
| JP 9-262475 A | 07.10.1997 | (Family: none) | |
| JP 9-263554 A | 07.10.1997 | (Family: none) | |
| JP 11-300207 A | 02.11.1999 | (Family: none) | |
| JP 2000-26343 A | 25.01.2000 | EP 955284 A1<br>paragraph [0016],<br>examples, claims<br>CN 1235951 A<br>KR 10-1999-0083558 A | |
| JP 10-101601 A | 21.04.1998 | (Family: none) | |
| JP 2002-145813 A | 22.05.2002 | (Family: none) | |
| WO 2007/105421 A1 | 20.09.2007 | JP 2007-230934 A<br>KR 10-2008-0100190 A<br>CN 101395111 A | |
| WO 2020/090756 A1 | 07.05.2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H0380136 B
- JP 3041414 B
- JP 2001079395 A
- JP 3901233 B
- JP H08225473 A
- JP 2003190786 A


### Non-patent literature cited in the description


- Quarterly Chemical Review. Chemistry of Polyacids. 1993
- New Experimental Chemistry 8, Synthesis of Inorganic Compound (III). Maruzen Co., Ltd, 20 August 1984, 1413